(19) Europäisches Patentamt
European Patent Office
Office européen des brevets

(11) **EP 0 665 211 B1**

(12) **FASCICULE DE BREVET EUROPEEN**

(45) Date de publication et mention
de la délivrance du brevet:
**03.09.1997 Bulletin 1997/36**

(51) Int. Cl.$^6$: **C07C 57/155**, C07C 51/56

(21) Numéro de dépôt: **95400114.5**

(22) Date de dépôt: **20.01.1995**

(54) **Procédé de préparation d'anhydride citraconique**

Verfahren zur Herstellung von Zitrakonsäureanhydrid

Process for preparing citraconic anhydride

(84) Etats contractants désignés:
**BE CH DE FR GB IE IT LI NL SE**

(30) Priorité: **28.01.1994 FR 9400938**

(43) Date de publication de la demande:
**02.08.1995 Bulletin 1995/31**

(73) Titulaire: **RHONE-POULENC CHIMIE**
**92408 Courbevoie Cédex (FR)**

(72) Inventeurs:
• **Tirel, Philippe-Jean**
**F-69360 Communay (FR)**
• **Sigismondi, Alain**
**F-69007 Lyon (FR)**
• **Alas, Michel**
**F-79500 Melle (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**92408 Courbevoie Cédex (FR)**

(56) Documents cités:
**EP-A- 0 495 544**

• **JOURNAL OF POLYMER SCIENCE, POLYMER**
**CHEMISTRY EDITION, vol.19, 1981, NEW YORK**
**US pages 2243 - 2253 A.V.GALANTI ET AL.**
**'MECHANISM OF AMINE CATALYZED**
**ISOMERIZATION OF ITACONIC ANHYDRIDE TO**
**CITRACONIC ANHYDRIDE: CITRACONAMIC**
**ACID FORMATION.'**

## Description

La présente invention concerne un nouveau procédé de préparation de l'anhydride citraconique. Plus précisément, l'invention a trait à un procédé de préparation de l'anhydride citraconique, à partir de l'acide itaconique.

Il est connu selon US 2 966 498, de préparer l'anhydride citraconique par chauffage d'acide itaconique, à une température comprise entre 165°C et 190°C, en présence d'un catalyseur choisi dans le groupe formé par le dihydrogénophosphate d'un métal alcalin et le sulfate de métal alcalin. Ledit procédé est intéressant du fait que l'anhydride citraconique est obtenu en une seule étape mais il souffre de plusieurs inconvénients. La régulation de la température est délicate à assurer. L'élimination de l'eau formée, en faible quantité au cours de la réaction est difficile à réaliser et sa distillation entraîne également de l'anhydride citraconique ce qui conduit à une perte du produit obtenu. De plus, dans ce type de procédé, la polymérisation de l'anhydride citraconique est favorisée. Enfin, la présence d'un catalyseur tel que précité provoque un blindage du réacteur dès lors que la quantité de catalyseur mise en oeuvre est importante.

D'autres voies d'accès à l'anhydride citraconique sont décrites dans la littérature. Ainsi, il est possible de préparer l'anhydride citraconique par isomérisation de l'anhydride itaconique. A. Galanti et al [Journal of Polymer Science, Polymer Chemistry Edition, 19, 2243-2253 (1981)] ont décrit l'isomérisation de l'anhydride itaconique, en présence d'une amine. La mise en oeuvre de cette réaction implique de partir d'une matière première, l'anhydride itaconique qui doit être lui-même préparé à partir d'acide itaconique ce qui implique donc, forcément que le procédé comporte deux étapes.

L'objectif de la présente invention est de fournir un procédé, en une seule étape, qui ne présente pas les inconvénients précités.

Il a maintenant été trouvé et c'est ce qui constitue l'objet de la présente invention, un procédé de préparation d'anhydride citraconique, à partir d'acide itaconique, caractérisé par le fait qu'il consiste à chauffer l'acide itaconique, en présence d'un catalyseur au moins partiellement organique, de type acido-basique, ayant un pKa compris entre 4 et 10.

La caractéristique du procédé de l'invention réside dans le choix du catalyseur qui permet d'obtenir directement l'anhydride citraconique, à partir de l'acide itaconique.

Un premier impératif qui préside au choix du catalyseur est que ce soit un sel ayant un pKa compris entre 4 et 10, de préférence compris entre 5 et 9 : le pKa étant défini comme le cologarithme de la constante de dissociation de l'acide mesuré, en milieu aqueux, à 25°C.

Une autre caractéristique du catalyseur utilisé dans le procédé de l'invention est qu'il soit fusible ou fondu, dans la masse réactionnelle. Il importe que son point de fusion soit, de préférence, inférieur à 200°C, et plus préférentiellement inférieur ou égal à 180°C.

Comme mentionné précédemment, le catalyseur associe un acide et une base, avec au moins l'un des deux qui est un composé organique.

Le catalyseur peut être un sel résultant de la réaction d'un acide et d'une base, qui peut êre préparé extemporanément.

Pour former le sel, on met généralement en oeuvre, les quantités d'acide et de base requises par la stoechiométrie.

Il peut également s'agir d'un sel préparé in situ, par addition d'un acide et d'une base ou bien par addition seulement d'une base, le sel obtenu résultant de la réaction du réactif acide itaconique et de la base.

Le catalyseur résulte donc de la réaction d'un acide minéral ou organique et d'une base.

Comme exemples non limitatifs de tels acides, on peut citer les acides halogénés tels que l'acide chlorhydrique, l'acide bromhydrique, l'acide fluorhydrique; les oxyacides halogénés ou non tels que l'acide sulfurique, l'acide pyrosulfurique, l'acide perchlorique, l'acide phosphorique; les acides sulfoniques halogénés ou non, tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluénesulfoniques, les acides naphtalènesulfoniques et les acides naphtalènedisulfoniques.

Parmi ces acides, on utilisera de préférence l'acide chlorhydrique, l'acide bromhydrique, l'acide trifluorométhanesulfonique, l'acide paratoluènesulfonique, l'acide méthanesulfonique.

L'acide itaconique peut également intervenir dans la préparation du catalyseur ou d'autres acides carboxyliques non volatils dans les conditions réactionnelles.

Pour ce qui est de la base, il s'agit d'un composé donneur d'un doublet électronique.

On peut faire appel à des bases azotées primaires, secondaires ou tertiaires et l'on peut citer plus particulièrement :

- l'ammoniac ;
- des amines primaires telles que la n-propylamine, l'isopropylamine, l'isobutylamine, la n-butylamine, la tertiobutylamine, la n-pentylamine, la 2-méthyl butylamine, la 3-méthyl butylamine, la n-hexylamine, la 2-éthyl hexylamine, l'aniline, la laurylamine, la cyclohexylamine, la cyclopentylamine, la benzylamine, la guanidine, l'acétamidine, l'éthanolamine, l'éthylènediamine, l'hexaméthylènediamine, la N-aminoéthylpyrrolidine, la pyrazoline, la N-aminomorpholine, la N-aminopipéridine, la tétraéthylènepentamine ;

- des amines secondaires telles que la dibutylamine, la dipropylamine, la méthylpropylamine, la méthylbutylamine, la méthylisobutylamine, la méthyltertiobutylamine, la méthylbenzylamine, la ditertiobutylamine, la diéthanolamine, la 1-méthyl cyclo-pentylamine, la 1-méthyl cyclohexylamine, la dicyclohexylamine, la morpholine, l'imidazole, la pyrrolidine, l'imidazolidine, la pipérazine, l'indole ;
- des amines tertiaires telles que la triéthylamine, la tributylamine, la diméthylaniline, la pyridine, la pyrazine, la triéthanolamine, la tris(3,6-dioxa heptyl)amine, le 1,8-diaza (5,4,0)bicyclo 7-undécène.

Parmi tous les composés azotés précités, on préfère choisir les bases tertiaires azotées hétérocycliques saturées ou insaturées, et préférentiellement, la pyridine ou la pyrazine. On peut également faire appel aux dérivés de substitution ($\alpha$-picoline, $\beta$-picoline).

Un autre groupe de bases convenant à la mise en oeuvre du procédé de l'invention est constitué par les phosphines.

Les trialcoyl- et triarylphosphines sont utilisées de préférence. On peut citer notamment, la triméthylphosphine, la triéthylphosphine, la tri-n-propylphosphine, la tri-n-butylphosphine, la tricyclohexylphosphine, la triphénylphosphine, la tritolylphosphine.

On donne ci-après des exemples de catalyseurs convenant tout à fait bien à la mise en oeuvre de l'invention :

- le tosylate de pyridinium,
- l'itaconate d'ammonium,
- l'itaconate de pyridinium,
- le chlorhydrate de pyridinium,
- le bromhydrate de phosphinium.

La quantité de catalyseur utilisée dans le procédé de l'invention est telle qu'il représente de 0,1 à 30 %, de préférence de 1 à 4 % du poids de l'acide itaconique.

La réaction peut être conduite en l'absence de tout solvant ou bien en présence d'un solvant organique.

Le choix du solvant est déterminé en fonction de son aptitude à solubiliser l'acide itaconique de départ.

De plus, il doit être inerte dans les conditions réactionnelles.

Il est préférable d'utiliser comme solvant organique, un liquide formant avec l'eau, un azéotrope binaire dont le point d'ébullition est généralement d'au moins 130°C. Ledit solvant organique est choisi de telle façon que l'azéotrope binaire qu'il forme avec l'eau ait un point d'ébullition inférieur :

- au point d'ébullition de l'acide itaconique,
- au point d'ébullition de l'azéotrope binaire que l'acide itaconique serait suceptible de former, soit avec l'eau, soit avec le solvant organique lui-même.

Enfin, on choisit de préférence, le solvant organique de telle façon qu'il ne forme pas d'azéotrope ternaire avec l'acide itaconique et l'eau, afin de limiter les pertes dudit acide.

Les solvants organiques pouvant être utilisés dans le cadre du procédé de l'invention ont de préférence un point d'ébullition compris entre 110°C et 200°C, de préférence de 130°C à 170°C.

A titre d'exemples non limitatifs, on peut citer :

- les hydrocarbures aliphatiques et plus particulièrement les paraffines tels que notamment, l'octane, l'isooctane, le nonane, le décane, l'undécane, le tétradécane ; les hydrocarbures aromatiques comme notamment le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso®,
- les hydrocarbures aliphatiques chorés comme, par exemple, le 1,1,2-trichloroéthane, le pentachloroéthane, le 1-iodo-2-méthylpropane, le 1-chlorohexane, le 1-chloro-2-éthylhexane ; les hydrocarbures aromatiques chorés et plus particulièrement, le chlorobenzène, les chlorotoluènes,
- les éthers et plus particulièrement les éthers aliphatiques comme l'éther butylique, l'éther isobutylique, l'éthylhexyléther, le 1-butoxy-2-méthoxyéthane, le 1,1-diéthoxybutane, l'éther amylique, l'éther isoamylique, le dipropoxyméthane ; les éthers aromatiques comme le phénylpropyléther, l'oxyde de mésityle,
- les composés nitrés tels que le nitropropane, le nitrobenzène.

Parmi les solvants précités, on choisit préférentiellement les hydrocarbures aromatiques, et plus spécialement le cumène et le pseudocumène.

Dans le cas où le solvant organique présente une température d'ébullition trop basse, il est possible de conduire le procédé de l'invention, sous pression.

EP 0 665 211 B1

La température à laquelle s'effectue la réaction est généralement la température de reflux du mélange réactionnel.

La température à laquelle est conduite la réaction varie entre 130°C et 180°C, de préférence entre 150°C et 170°C.

Lorsque l'eau formée par la réaction est éliminée par distillation azéotropique, cette élimination peut se faire de manière continue ou discontinue.

L'eau de l'azéotrope binaire eau-solvant organique peut être éliminée, soit par passage dudit azéotrope sur un solide adsorbant l'eau tel que par exemple, les tamis moléculaires avant recyclage, soit par décantation.

Après décantation de la couche de l'azéotrope hétérogène contenant la majeure partie de l'eau, on peut recycler l'autre couche dans le mélange réactionnel.

La concentration de l'acide itaconique dans le mélange réactionnel (acide itaconique + solvant organique) n'est pas critique. Habituellement, l'acide mis en oeuvre représente de 10 à 100 %, de préférence de 10 à 50 % du poids du solvant réactionnel.

Selon une variante préférée du procédé de l'invention, on conduit le procédé de l'invention, sous atmosphère contrôlée de gaz inertes. On peut établir une atmosphère de gaz rares, de préférence l'argon mais il est plus économique de faire appel à l'azote.

D'un point de vue pratique, la réaction est aisément mise en oeuvre. L'ordre d'introduction des réactifs n'est pas critique. Généralement, on charge le solvant réactionnel, l'acide itaconique et le catalyseur. On chauffe à la température souhaitée et l'on élimine l'eau formée par distillation azéotropique tout au long de la réaction.

La mise en oeuvre du catalyseur de l'invention est particulièrement avantageuse.

Dans une variante préférée, le catalyseur forme, lors du refroidissement, un système bi-phasique, ce qui permet de le séparer très aisément, notamment par décantation.

Après séparation du catalyseur, on élimine le solvant organique, de préférencé, par distillation et l'on récupère l'anhydride citraconique par les moyens classiques utilisés dans ce domaine technique, de préférence, par distillation.

Les exemples qui suivent, illustrent l'invention, sans pour autant la limiter.

Dans les exemples, les abréviations ont la signification suivante ;

$$TT = \frac{\text{nombre de moles d'acide itaconique transformées}}{\text{nombre de moles d'acide itaconique introduites}} \%$$

$$RR = \frac{\text{nombre de moles d'anhydride citraconique formées}}{\text{nombre de moles d'acide itaconique introduites}} \%$$

Exemple 1

Dans un réacteur de 50 ml muni d'une agitation mécanique bipale, maintenu sous azote, on charge :

- 20 ml de pseudocumène,
- 2 g d'acide itaconique,
- 0,5 g de p-toluènesulfonate de pyridinium.

Le mélange est agité et chauffé à 166°C, pendant 3 heures, sous pression atmosphérique.

L'eau produite est éliminée par distillation azéotropique avec le pseudocumène, tout au long de la réaction.

On dose le produit obtenu par chromatographie liquide haute performance.

Les résultats obtenus sont les suivants :

- TT = 100 %
- RR = 90 %

Exemple 2

On reproduit l'exemple 1 à la différence près que le p-toluènesulfonate de pyridinium est remplacé par l'itaconate de pyridinium, préparé in situ par ajout de 0,5 g de pyridine.

Après dosage, les résultats obtenus sont les suivants :

- TT = 95 %
- RR = 87%

4

Exemple 3

On reproduit l'exemple 1 à la différence près que le p-toluènesulfonate de pyridinium est remplacé par l'itaconate de pyrazinium, préparé in situ par ajout de 0,5 g de pyrazine.

Après dosage, les résultats obtenus sont les suivants :

- TT = 100%
- RR = 80%

Exemple 4

On reproduit l'exemple 1 à la différence près que le p-toluènesulfonate de pyridinium est remplacé par 0,5 g de chlorhydrate de pyridinium.

Après dosage, les résultats obtenus sont les suivants :

- TT = 95 %
- RR = 88 %

Exemple 5

Dans un réacteur de 50 cm$^3$, muni d'une agitation mécanique bipale, on charge :

- 9 ml de pseudocumène,
- 10 g d'acide itaconique,
- 0,7 g de p-toluènesulfonate de pyridinium.

Le mélange est agité et chauffé à 166°C pendant 1 heure.
L'eau produite est éliminée par distillation azéotropique.
Après dosage, les résultats obtenus sont les suivants :

- TT = 99 %
- RR = 90 %

Exemple 6

On reproduit l'exemple 6 mais en mettant en oeuvre 0,4 g de p-toluène sulfonate de pyridinium à la place de 0,7 g.
Après dosage, les résultats obtenus sont les suivants :

- RR = 85%

**Revendications**

1. Procédé de préparation d'anhydride citraconique à partir d'acide itaconique, caractérisé par le fait qu'il consiste à chauffer l'acide itaconique, en présence d'un catalyseur au moins partiellement organique, de type acido-basique, ayant un pKa compris entre 4 et 10.

2. Procédé selon la revendication 1 caractérisé par le fait que le catalyseur est un sel ayant un pka compris entre 5 et 9.

3. Procédé selon l'une des revendications 1 et 2 caractérisé par le fait que le catalyseur a un point de fusion inférieur à 200°C, et de préférence, inférieur ou égal à 180°C.

4. Procédé selon l'une des revendications 1 à 3 caractérisé par le fait que le catalyseur est un sel résultant de la réaction d'un acide ou d'une base, préparé extemporanément ou in situ.

5. Procédé selon l'une des revendications 1 à 3, caractérisé par le fait que le catalyseur est un sel préparé in situ par réaction de l'acide itaconique et d'une base.

6. Procédé selon l'une des revendications 1 à 5 caractérisé par le fait que le catalyseur est préparé à partir d'un acide choisi parmi les acides halogénés tels que l'acide chlorhydrique, l'acide bromhydrique, l'acide fluorhydrique ; les oxyacides halogénés ou non tels que l'acide sulfurique, l'acide pyrosulfurique, l'acide perchlorique, l'acide phosphorique les acides sulfoniques halogénés ou non, tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques les acides carboxyliques, de préférence l'acide itaconique.

7. Procédé selon l'une des revendications 1 à 6 caractérisé par le fait que l'acide est l'acide chlorhydrique, l'acide bromhydrique, l'acide trifluorométhanesulfonique, l'acide paratoluènesulfonique, l'acide méthanesulfonique ou l'acide itaconique.

8. Procédé selon l'une des revendications 1 à 7 caractérisé par le fait que le catalyseur est préparé à partir d'une base choisie parmi :

- l'ammoniac ;
- les amines primaires telles que la n-propylamine, l'isopropylamine, l'isobutylamine, la n-butylamine, la tertiobutylamine, la n-pentylamine, la 2-méthyl butylamine, la 3-méthyl butylamine, la n-hexylamine, la 2-éthyl hexylamine, l'aniline, la laurylamine, la cyclohexylamine, la cyclopentylamine, la benzylamine, la guanidine, l'acétamidine, l'éthanolamine, l'éthylènediamine, l'hexaméthylènediamine, la N-aminoéthylpyrrolidine, la pyrazoline, la N-aminomorpholine, la N-aminopipéridine, la tétraéthylènepentamine ;
- les amines secondaires telles que la dibutylamine, la dipropylamine, la méthylpropylamine, la méthylbutylamine, la méthylisobutylamine, la méthyltertiobutylamine, la méthylbenzylamine, la ditertiobutylamine, la 1-méthyl cyclopentylamine, la 1-méthyl cyclohexylamine, la diéthanolamine, la dicyclohexylamine, la morpholine, l'imidazole, la pyrrolidine, l'imidazolidine, la pipérazine, l'indole ;
- les amines tertiaires telles que la triéthylamine, la tributylamine, la diméthylaniline, la pyridine, la pyrazine, la triéthanolamine, la tris(3,6-dioxa heptyl)amine, le 1,8-diaza (5,4,0)bicyclo 7-undécène.
- les trialcoyl- et triarylphosphines, de préférence notamment, la triméthylphosphine, la triéthylphosphine, la tri-n-propylphosphine, la tri-n-butylphosphine, la tricyclohexylphosphine, la triphénylphosphine, la tritolyl-phosphine.

9. Procédé selon la revendication 8 caractérisé par le fait que la base est une base azotée hétérocyclique, saturée ou insaturée, éventuellement substituée, de préférence, la pyridine ou la pyrazine.

10. Procédé selon l'une des revendications 1 à 9 caractérisé par le fait que le catalyseur est choisi parmi :

- le tosylate de pyridinium,
- l'itaconate d'ammonium,
- l'itaconate de pyridinium,
- le chlorhydrate de pyridinium,
- le bromhydrate de phosphinium.

11. Procédé selon l'une des revendications 1 à 10 caractérisé par le fait que la quantité de catalyseur représente de 0,1 à 30 %, de préférence de 1 à 4 % du poids de l'acide itaconique.

12. Procédé selon l'une des revendications 1 à 11 caractérisé par le fait que la réaction est conduite dans un solvant organique ayant un point d'ébullition compris entre 110°C et 200°C, de préférence de 130°C à 170°C.

13. Procédé selon l'une des revendications 1 à 12 caractérisé par le fait que le solvant organique est choisi parmi :

- les hydrocarbures aliphatiques et plus particulièrement les paraffines tels que notamment, l'octane, l'isooctane, le nonane, le décane, l'undécane, le tétradécane ; les hydrocarbures aromatiques comme notamment le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso®,
- les hydrocarbures aliphatiques chorés comme, par exemple, le 1,1,2-trichloroéthane, le pentachloroéthane, le 1-iodo-2-méthylpropane, le 1-chlorohexane, le 1-chloro-2-éthylhexane ; les hydrocarbures aromatiques chorés et plus particulièrement, le chlorobenzène, les chlorotoluènes,
- les éthers et plus particulièrement les éthers aliphatiques comme l'éther butylique, l'éther isobutylique, l'éthylhexyléther, le 1-butoxy-2-méthoxyéthane, le 1,1-diéthoxybutane, l'éther amylique, l'éther isoamylique, le dipro-

EP 0 665 211 B1

poxyméthane ; les éthers aromatiques comme le phénylpropyléther, l'oxyde de mésityle,
- les composés nitrés tels que le nitropropane, le nitrobenzène.

**14.** Procédé selon la revendication 13 caractérisé par le fait que le solvant organique est un hydrocarbure aromatique, de préférence, le cumène ou le pseudocumène.

**15.** Procédé selon l'une des revendications 1 à 14 caractérisé par le fait que la température de la réaction varie entre 130°C et 180°C, de préférence entre 150°C et 170°C.

**Claims**

1. A process for the preparation of citraconic anhydride from itaconic acid, characterized in that it consists of heating itaconic acid in the presence of a catalyst which is at least partially organic, acidobasic, and with a $pK_a$ which is in the range 4 to 10.

2. A process according to claim 1, characterized in that the catalyst is a salt with a $pK_a$ which is in the range 5 to 9.

3. A process according to claim 1 or claim 2, characterized in that the catalyst has a melting point of less than 200°C, preferably less than or equal to 180°C.

4. A process according to any one of claims 1 to 3, characterized in that the catalyst is a salt resulting from the reaction of an acid or a base, prepared extemporaneously or in situ.

5. A process according to any one of claims 1 to 3, characterized in that the catalyst is a salt prepared in situ by reacting itaconic acid and a base.

6. A process according to any one of claims 1 to 5, characterized in that the catalyst is prepared from an acid selected from: halogenated acids such as hydrochloric acid, hydrobromic acid, or hydrofluoric acid; oxyacids which are halogenated or not halogenated, such as sulphuric acid, pyrosulphuric acid, perchloric acid, or phosphoric acid; sulphonic acids which are halogenated or not halogenated, such as fluorosulphonic acid, chlorosulphonic acid, trifluoromethanesulphonic acid, methanesulphonic acid, ethanesulphonic acid, ethanedisulphonic acid, benezenesulphonic acid, benzenedisulphonic acids, toluenesulphonic acids, naphthalenesulphonic acids and naphthalenedisulphonic acids; and carboxylic acids, preferably itaconic acid.

7. A process according to any one of claims 1 to 6, characterized in that the acid is hydrochloric acid, hydrobromic acid, trifluoromethanesulphonic acid, para-toluenesulphonic acid, methanesulphonic acid or itaconic acid.

8. A process according to any one of claims 1 to 7, characterized in that the catalyst is prepared from a base selected from:

   • ammonia;
   • primary amines such as n-propylamine, isopropylamine, isobutylamine, n-butylamine, tertiobutylamine, n-pentylamine, 2-methylbutylamine, 3-methyl butylamine, n-hexylamine, 2-ethylhexylamine, aniline, laurylamine, cyclohexylamine, cyclopentylamine, benzylamine, guanidine, acetamidine, ethanolamine, ethylenediamine, hexamethylenediamine, N-aminoethylpyrrolidine, pyrazoline, N-aminomorpholine, N-aminopiperidine, and tetraethylenepentamine;
   • secondary amines such as dibutylamine, dipropylamine, methylpropylamine, methylbutylamine, methylisobutylamine, methyltertiobutylamine, methylbenzylamine, ditertiobutylamine, 1-methylcyclopentylamine, 1-methylcyclohexylamine, diethanolamine, dicyclohexylamine, morpholine, imidazole, pyrrolidine, irnidazolidine, piperazine, and indole;
   • tertiary amines such as triethylamine, tributylamine, dimethylaniline, pyridine, pyrazine, triethanolamine, tris(3,6-dioxaheptyl)amine, and 1,8-diaza(5,4,0)bicyclo-7-updecene;
   • trialkoyl- and triaryl-phosphines, preferably trimethylphosphine, triethylphosphine, tri-n-propylphosphine, tri-n-butylphosphine, tricyclohexylphosphine, triphenylphosphine, and tritolylphosphine.

9. A process according to claim 8, characterized in that the base is a saturated or unsaturated heterocyclic nitrogen-containing base which may be substituted, preferably pyridine or pyrazine.

10. A process according to any one of claims 1 to 9, characterized in that the catalyst is selected from :

7

- pyridinium tosylate;
- ammonium itaconate;
- pyridinium itaconate;
- pyridinium hydrochloride;
- phosphonium hydrobromide.

11. A process according to any one of claims 1 to 10, characterized in that the catalyst is present in quantities of 0.1% to 30%, preferably 1% to 4%, of the weight of the itaconic acid.

12. A process according to any one of claims 1 to 11, characterized in that the reaction is carried out in an organic solvent with a boiling point which is in the range 110°C to 200°C, preferably in the range 130°C to 170°C.

13. A process according to any one of claims 1 to 12, characterized in that the organic solvent is selected from:

- aliphatic hydrocarbons, more particularly paraffins such as octane, isooctane, nonane, decane, undecane, or tetradecane; aromatic hydrocarbons such as toluene, xylenes, ethylbenzene, diethylbenzenes, trimethylbenzenes, cumene, or pseudocumene, or petroleum cuts constituted by a mixture of alkylbenzenes, in particular Solvesso® type cuts;
- chlorinated aliphatic hydrocarbons such as 1,1,2-trichloroethane, pentachloroethane, 1-iodo-2-methylpropane, 1-chlorohexane, 1-chloro-2-ethylhexane; and chlorinated aromatic hydrocarbons, in particular chlorobenzene and chlorotoluenes;
- ethers, more particularly aliphatic ethers such as butyl ether, isobutyl ether, ethylhexylether, 1-butoxy-2-methoxyethane, 1,1-diethoxybutane, amyl ether, isoamyl ether, dipropoxymethane; and aromatic ethers such as phenylpropylether, mesityl oxide;
- nitro compounds such as nitropropane, nitrobenzene.

14. A process according to claims 13, characterized in that the organic solvent is an aromatic hydrocarbon, preferably cumene or pseudocumene.

15. A process according to any one of claims 1 to 14, characterized in that the reaction temperature is between 130°C and 180°C, preferably between 150°C and 170°C.

**Patentansprüche**

1. Verfahren zur Herstellung von Zitrakonsäureanhydrid ausgehend von Itakonsäure, dadurch gekennzeichnet, daß man die Itakonsäuure in Gegenwart eines zumindest teilweise organischen Katalysators vom Säure/Base-Typ mit einem $pK_a$-Wert zwischen 4 und 10 erhitzt.

2. Verfahren nach Anspruch 1, dadurch gekennzeichnet, daß der Katalysator ein Salz mit einem $pK_a$-Wert zwischen 5 und 9 ist.

3. Verfahren nach Anspruch 1 oder 2, dadurch gekennzeichnet, daß der Katalysator einen Schmelzpunkt unterhalb von 200 °C, vorzugsweise von unterhalb oder gleich 180 °C, aufweist.

4. Verfahren nach einem der Ansprüche 1 bis 3, dadurch gekennzeichnet, daß der Katalysator ein Salz ist, das aus der Reaktion einer Säure oder einer Base stammt und unmittelbar zuvor oder in situ hergestellt ist.

5. Verfahren nach einem der Ansprüche 1 bis 3, dadurch gekennzeichnet, daß der Katalysator ein Salz ist, das in situ mittels Reaktion von Itakonsäure und einer Base hergestellt worden ist.

6. Verfahren nach einem der Ansprüche 1 bis 5, dadurch gekennzeichnet, daß der Katalysator ausgehend von einer Säure hergestellt wird, die ausgewählt ist aus halogenhaltigen Säuren wie Chlorwasserstoffsäure, Bromwasserstoffsäure, Fluorwasserstoffsäure; halogenierten oder nichthalogenierten Oxysäuren wie Schwefelsäure, Pyroschwefelsäure, Perchlorsäure, Phosphorsäure; halogenierten oder nichthalogenierten Sulfonsäuren wie Fluorsulfonsäure, Chlorsulfonsäure oder Trifluormethansulfonsäure, Methansulfonsäure, Ethansulfonsäure, Ethandisulfonsäure, Benzolsulfonsäure, Benzoldisulfonsäuren, Toluolsulfonsäuren, Naphthalinsulfonsäuren und Naphthalindisulfonsäuren; Carbonsäuren, vorzugsweise Itakonsäure.

7. Verfahren nach einem der Ansprüche 1 bis 6, dadurch gekennzeichnet, daß die Säure Chlorwasserstoffsäure, Bromwasserstoffsäure, Trifluormethansulfonsäure, Paratoluolsulfonsäure, Methansulfonsäure oder Itakonsäure ist.

8. Verfahren nach einem der Ansprüche 1 bis 7, dadurch gekennzeichnet, daß der Katalysator hergestellt ist ausgehend von einer Base, die ausgewählt ist aus:

- Ammoniak;
- primären Aminen, wie n-Propylamin, Isopropylamin, Isobutylamin, n-Butylamin, Tertiobutylamin, n-Pentylamin, 2-Methylbutylamin, 3-Methylbutylamin, n-Hexylamin, 2-Ethylhexylamin, Anilin, Laurylamin, Cyclohexylamin, Cyclopentylamin, Benzylamin, Guarudin, Acetamidin, Ethanolamin, Ethylendiamin, Hexamethylendiamin, N-Aminoethylpyrrolidin, Pyrazolin, N-Aminomorpholin, N-Aminopiperidin, Tetraethylenpentamin;
- sekundären Aminen wie Dibutylamin, Dipropylamin; Methylpropylamin, Methylbutylamin, Methylisobutylamin, Methyltertiobutylamin, Methylbenzylamin, Ditertiobutylamin, 1-Methylcyclopentylamin, 1-Methylcyclohexylamin, Diethanolamin, Dicyclohexylamin, Morpholin, Imidazol, Pyrrolidin, Imidazolidin, Piperazin, Indol;
- tertiären Aminen wie Triethylamin, Tributylamin, Dimethylanilin, Pyridin, Pyrazin, Triethanolamin, Tris-(3,6-dioxaheptyl)amin, 1,8-Diaza[5.4.0]-bicyclo-7-undecen;
- Trialkyl- und Triarylphosphinen, vorzugsweise insbesondere Trimethylphosphin, Triethylphosphin, Tri-n-propylphosphin, Tri-n-butylphosphin, Tricyclohexylphosphin, Triphenylphosphin, Tritolylphosphin.

9. Verfahren nach Anspruch 8, dadurch gekennzeichnet, daß die Base eine stickstoffhaltige heterocyclische Base ist, die gesättigt oder ungesättigt und gegebenenfalls substituiert ist, vorzugsweise Pyridin oder Pyrazin.

10. Verfahren nach einem der Ansprüche 8 bis 9, dadurch gekennzeichnet, daß der Katalysator ausgewählt ist aus:

- Pyridiniumtosylat,
- Ammoniumitakonat,
- Pyridiniumitakonat,
- Pyridiniumchlorhydrat,
- Phosphiniumbromhydrat.

11. Verfahren nach einem der Ansprüche 1 bis 10, dadurch gekennzeichnet, daß die Menge an Katalysator 0,1 bis 30 %, vorzugsweise 1 bis 4 %, des Gewichts der Itakonsäure darstellt.

12. Verfahren nach einem der Ansprüche 1 bis 11, dadurch gekennzeichnet, daß die Reaktion in einem organischen Lösungsmittel mit einem Siedepunkt zwischen 110 °C und 200 °C, vorzugsweise von 130 °C bis 170 °C, durchgeführt wird.

13. Verfahren nach einem der Ansprüche 1 bis 12, dadurch gekennzeichnet, daß das organische Lösungsmittel ausgewählt ist:

- aliphatischen Kohlenwasserstoffen und insbesondere Paraffinen wie insbesondere Octan, Isooctan, Nonan, Decan, Undecan, Tetradecan;
- aromatischen Kohlenwasserstoffen wie insbesondere Toluol, Xylolen, Ethylbenzol, Diethylbenzolen, Trimethylbenzolen, Cumol, Pseudocumol, Kohlenwasserstoffreaktionen die aus einer Mischung von Alkylbenzolen bestehen, insbesondere Fraktionen vom Typ Solvesso®,
- chlorierten aliphatischen Kohlenwasserstoffen, wie z.B. 1,1,2-Trichlorethan, Pentachlorethan, 1-Iod-2-methylpropan, 1-Chlorhexan, 1-Chlor-2-ethylhexan; chlorierten aromatischen Kohlenwasserstoffen und insbesondere Chlorbenzol und Chlortoluolen,
- Ethern und insbesondere aliphatischen Ethern wie Butylether, Isobutylether, Ethylhexylether, 1-Butoxy-2-methoxyethan, 1,1-Diethoxybutan, Amylethern, Isoamylethern, Dipropoxymethan; aromatischen Ethern wie Phenylpropylether und Mesityloxid,
- nitrierten Verbindungen wie Nitropropan und Nitrobenzol.

14. Verfahren nach Anspruch 13, dadurch gekennzeichnet, daß das organische Lösungsmittel ein aromatischer Kohlenwasserstoff ist, vorzugsweise Cumol oder Pseudocumol.

15. Verfahren nach einem der Ansprüche 1 bis 14, dadurch gekennzeichnet, daß die Reaktionstemperatur zwischen 130 °C und 180 °C, vorzugsweise zwischen 150 °C und 170 °C, variiert.